(19) Europäisches Patentamt / European Patent Office / Office européen des brevets

(11) **EP 1 485 717 B1**

(12) **EUROPÄISCHE PATENTSCHRIFT**

(45) Veröffentlichungstag und Bekanntmachung des Hinweises auf die Patenterteilung:
**14.02.2007 Patentblatt 2007/07**

(21) Anmeldenummer: **03718618.6**

(22) Anmeldetag: **07.03.2003**

(51) Int Cl.:
***G01N 33/536*** *(2006.01)*

(86) Internationale Anmeldenummer:
**PCT/DE2003/000745**

(87) Internationale Veröffentlichungsnummer:
**WO 2003/079011 (25.09.2003 Gazette 2003/39)**

(54) **VERFAHREN ZUR QUANTITATIVEN BESTIMMUNG MEHRERER ANALYTEN**

METHOD FOR QUANTITATIVELY DETERMINING A NUMBER OF ANALYTES

PROCEDE DE DETERMINATION QUANTITATIVE DE PLUSIEURS ANALYTES

(84) Benannte Vertragsstaaten:
**AT BE BG CH CY CZ DE DK EE ES FI FR GB GR HU IE IT LI LU MC NL PT RO SE SI SK TR**

(30) Priorität: **16.03.2002 DE 10211818**

(43) Veröffentlichungstag der Anmeldung:
**15.12.2004 Patentblatt 2004/51**

(73) Patentinhaber: **PES GESELLSCHAFT FÜR MEDIZINISCHE DIAGNOSESYSTEME MBH**
**04416 Leipzig-Markkleeberg (DE)**

(72) Erfinder:
• **KEMPIN, Uwe**
**4109 Leipzig (DE)**
• **KELLER, Thomas, A.**
**04275 Leipzig (DE)**

(74) Vertreter: **Pfeiffer, Eva**
**Patentanwälte**
**Riechelmann & Carlsohn**
**Wiener Strasse 91**
**01219 Dresden (DE)**

(56) Entgegenhaltungen:
**WO-A-96/19731          DE-A- 19 838 802**
**US-A- 6 083 763**

• **WIESE RICK ET AL: "Simultaneous multianalyte ELISA performed on a microarray platform." CLINICAL CHEMISTRY, Bd. 47, Nr. 8, August 2001 (2001-08), Seiten 1451-1457, XP002254091 ISSN: 0009-9147**

**Beschreibung**

Die Erfindung betrifft ein Verfahren zur quantitativen Bestimmung von Analyten. Sie betrifft insbesondere einen Affinitäts-Assay zur quantitativen Bestimmung mehrerer Analyten über spezifische Bindungen zwischen Analyten und deren Bindungspartnern.

Affinitäts-Assays wie beispielsweise Fluoreszenzimmunotests oder auch Fluoreszenzimmunosensoren werden seit längerer Zeit eingesetzt, um eine unbekannte Menge einer zu bestimmenden chemischen oder biochemischen Substanz (Analyt) zu quantifizieren.

Aus DE 196 28 002 sind Verfahren bekannt, mit denen mehrere Analyten gleichzeitig bestimmt werden können. Dazu sind in einem Aufnahmebereich an verschiedenen, aufeinanderfolgenden Orten unterschiedliche analytspezifische Antikörper für ein Sandwichassay immobilisiert. In einem Probenbehälter, in den die zu analysierende Probe gegeben wird, befinden sich unterschiedliche markierte analytspezifische Antikörper. Die Anzahl dieser Antikörper muß dabei der Anzahl der einzelnen Analyten entsprechen. Diese unterschiedlichen, markierten und analytspezifischen Antikörper binden an unterschiedlichen Orten des Aufnahmenreichs an, wobei der Ort der Anbindung von den zuvor immobilisierten analytspezifischen Antikörpern abhängt. Durch orts- und zeitaufgelöste Fluoreszenzlichtmessung können die Analyten quantifiziert werden. Dieses Verfahren erfordert somit für jeden Analyten genau einen analytspezifischen Antikörper und einen immobilisierten Antikörper, der wiederum selbst analytspezifisch ist. Für die Fluoreszenzlichtmessungen sind bei Verwendung unterschiedlicher markierter Antikörper in der Regel unterschiedliche Anregungswellen oder eine ortsaufgelöste Messung erforderlich, so daß eine entsprechend komplizierte Meßvorrichtung notwendig ist. Unterschiedliche markierte Antikörper sind in diesem Zusammenhang einerseits verschiedene Antikörper mit verschiedenen Farbstoffen und andererseits verschiedene Antikörper mit identischen Farbstoffen.

Aufgabe der Erfindung ist es, die Nachteile nach dem Stand der Technik zu beseitigen. Es soll insbesondere ein Verfahren angegeben werden, mit dem mehrere Analyten quantitativ mit verringertem Aufwand nachgewiesen werden können.

Diese Aufgabe wird durch die Merkmale der Ansprüche 1 und 18, 19 und 20 gelöst. Zweckmäßige Ausgestaltungen der Erfindungen ergeben sich aus den Merkmalen der Ansprüche 2 bis 17.

Nach Maßgabe der Erfindung ist ein Verfahren zum quantitativen Nachweis von mehreren unterschiedlichen Analyten vorgesehen, wobei die Anzahl n der Analyten zumindest 2 beträgt. Das Verfahren umfaßt die Schritte:

(a) Versetzen einer Probe, die die n Analyten enthält, mit zumindest einem markierten Detektor-Bindepartner unter Bildung von Detektor-Analyt-Komplexen, die aus jeweils einem Analyt- und mindestens einem Detektormolekül bestehen, wobei die Anzahl x der Detektor-Bindepartner kleiner oder gleich n - 1 ist, jeder der Detektor-Bindepartner an zumindest einen Analyten anbinden und zumindest einer der Detektor-Bindepartner an zumindest zwei Analyten anbinden kann;

(b) Anbinden der in Schritt (a) erhaltenen Detektor-Analyt-Komplexe an Fänger-Bindepartner unter Bildung von Detektor-Analyt-Fänger-Komplexen, wobei die Anzahl y der Fänger-Bindeparter der Anzahl n der Analyten entspricht und jeder Fänger-Bindepartner für zumindest einen Detektor-Analyten-Komplex spezifisch ist; und

(c) zeitabhängige Erfassung der Bildung der Detektor-Analyt-Fänger-Komplexe.

In dem vorgeschlagenen Verfahren wird somit im Gegensatz zum Stand der Technik eine Anzahl an Detektor-Bindungspartnern benötigt, die geringer als die Anzahl der gleichzeitig zu quantifizierenden Analyten ist. Vorzugsweise wird nur ein Detektor-Bindungspartner eingesetzt. Das gilt auch dann, wenn die Anzahl n der Analyten größer als zwei ist. Die Verwendung einer geringeren Zahl von Detektor-Bindungspartnern vereinfacht die Quantifizierung erheblich, da die zeitliche Erfassung der Detektor-Analyt-Fänger-Komplexe in Schritt (c) auf eine geringere Zahl von Detektor-Bindepartner im Vergleich zum Stand der Technik abgestimmt werden kann. Wird nur ein einziger Detektor-Bindungspartner verwendet, ist die Quantifizierung besonders einfach. Somit können Meßvorrichtungen mit einfacherem Aufbau zu diesem Zweck verwendet werden.

Die Detektor-Bindepartner können in Schritt (a) gleichzeitig oder nacheinander mit dem zu bestimmenden Analyten unter Bildung von Detektor-Analyt-Komplexen inkubiert werden.

Die in Schritt (a) gebildeten Detektor-Analyt-Komplexe können aus jeweils einem Analyt- und mindestens einem Detektormolekül bestehen, d. h. bei bestimmten Analyten (beispielsweise bei Proteinen wie Creatinkinase BB (CKBB), Immunoglobulin E (IgE) oder c-reaktivem Protein (CRP)) können auch mehrere identische Detektor-Bindepartner an einen Analyten binden.

Der Probe kann in Schritt (a) zumindest ein dritter Bindepartner zugesetzt werden, der spezifisch für einen Analyten ist und an einer Bindungsstelle des Analyten anbinden kann, die sich von der Bindungsstelle, an die die Detektor-Bindungspartner anbinden können, unterscheidet. Die so erhaltenen Detektor-Analyt-Komplexe, die den dritten Bindepartner umfassen, binden in Schritt (b) über den dritten Bindepartner an den immobilisierten, für den Analyten spezifischen Fänger-Bindepartner an.

In einer Ausführungsform bindet mindestens einer der Detektor-Analyt-Komplexe an mindestens zwei verschiedene Fänger-Bindepartner an. Es ist weiterhin möglich, daß zwei verschiedene Detektor-Analyt-Komplexe an einen Fänger-Bindepartner anbinden. Es ist dann möglich, quantitative Aussagen über eine Gruppe von Detektor-Analyt-Komplexen zu treffen.

Die Detektor-Bindungspartner sind vorzugsweise mit einem Fluoreszenz- oder Lumineszenzfarbstoff markiert.

Bei den Detektor-Bindepartnern, Fänger-Bindepartnern und dritten Bindepartnern, die in dem erfindungsgemäßen Verfahren verwendet werden können, kann es sich um Liganden, polyklonale und monoklonale Antikörper und deren Antigen bindende Fragmente; RNA; DNA; DNA/RNA-Derivate und deren Analoga, wie beispielsweise Aptamere; Proteine, wie beispielsweise Lectine; und Allergene handeln.

Vorzugsweise sind die Fänger-Bindepartner auf unterschiedlichen Oberflächenbereichen einer Oberfläche immobilisiert. Die Fänger-Bindepartner können an die Oberfläche direkt über hydrophobe/ionische Wechselwirkungen oder durch chemische Bindung gebunden werden. Es ist jedoch eine indirekte Anbindung über einen auf der Oberfläche befindlichen Immobilisierungsvermittler, wie beispielsweise einem Anti-Antikörper, Avidin oder ein Carrierprotein; oder durch chemische Bindung über einen Abstandhalter (Spacer) möglich.

Wird eine derartige Oberfläche mit Oberflächenbereichen verwendet, auf denen verschiedene Fänger-Bindepartner immobilisiert sind, und werden die Detektor-Analyt-Komplexe über die Oberfläche geleitet, dann bilden sich durch Anbinden der Detektor-Analyt-Komplexe an die verschiedenen Fängeroberflächenbereiche Detektor-Analyt-Fänger-Komplexe.

Die Bildung der Detektor-Analyt-Fänger-Komplexe wird in Abhängigkeit von der Zeit erfaßt. Dazu können beispielsweise Messungen an zumindest zwei unterschiedlichen Zeitpunkten oder durch Messungen am Endpunkt der Bildung der Detektor-Analyt-Fänger-Komplexe erfolgen. In beiden Fällen dient die Zunahme der zu bestimmenden Meßgröße, beispielsweise des Fluoreszenzlichtes, wenn mit Fluoreszenzfarbstoffen markierte Detektor-Bindepartner verwendet werden, zur Konzentrationsbestimmung. Es ist somit ausreichend, den Anstieg der Meßgröße zu bestimmen, um die Menge des Analyten in der Probe zu quantifizieren.

Für die Bestimmung kann beispielsweise ein optisches Sensorsystem, wie es in Anal. Chem. 1999, 5430-5435, in WO 98/41843 (DE 197 11 281) oder auch in WO 98/02732 beschrieben ist, verwendet werden. Hierbei werden die Detektor-Bindepartner mit einen Fluoreszenzfarbstoff markiert. Die zu bestimmende Probe, welche die verschiedenen zu bestimmenden Analyte enthält, wird hierbei mit den gewählten Detektor-Bindepartnern inkubiert. Hierdurch bilden sich unterschiedliche Detektor-Analyt-Komplexe (Analyt1-Detektor1, Analyt2-Detektor1, [Analyt3-Detektor2, Analyt4-Detektor2, Analyt5-Detektor2,..) aus. Diese Lösung wird anschließend über die unterschiedlichen Oberflächenbereiche mit den immobilisierten Fänger-Bindepartnern geleitet, wodurch die Detektor-Analyt-Komplexe an die immobilisierten Fänger-Bindepartner anbinden. Für die Anregung der Fluoreszenzfarbstoffe der Detektor-Analyt-Komplexe, die an die Fänger gebunden sind, wird ein durch ein Laser erzeugtes evaneszentes Feld benutzt. Dieses evaneszente Feld wird durch totale interne Reflexion des Laser-Lichts an der Grenzfläche zwischen dem hochbrechenden Oberflächenmaterial (beispielsweise PMMA) und dem niedrig brechenden flüssigen Probenmaterial erzeugt. Die Intensität des evaneszenten Feldes im flüssigen Probenmaterial vermindert sich zum zunehmender Entfernung von der Grenzfläche exponentiell. Diese hat zur Folge, daß nur der fluoreszenzmarkierte Komplex, der einen Sandwich mit dem jeweiligen Fänger-Bindepartner ausbildet, Fluoreszenzlicht emittiert. Markierte Komplexe außerhalb des evaneszenten Feldes werden nicht mehr angeregt und geben daher keine Beitrag zum Fluoreszenzlicht. Da die Anzahl der Sandwich-Komplexe, d. h. der Detektor-Analyt-Fänger-Komplexe, die pro Zeiteinheit gebildet werden, direkt von der Konzentration der fluoreszenzmarkierten Immunkomplexe abhängig ist, ist durch die Bestimmung des zeitabhängigen Fluoreszenzsignals ein Maß für die Analyt-Konzentration in der Probe gegeben. Aus der Zunahme des Fluoreszenzsignals an den verschiedenen Oberflächenbereichen wird die Anfangssteigerung in mV/s bestimmt.

Mit einer bekannten Menge der interessierenden Analyten kann die Bestimmung kalibriert werden. Das Ergebnis der Bestimmung der verschiedenen Analyten der unbekannten Probe kann mit den Meßwerten aus den Kalibrierungen verglichen werden und stellt somit eine qualitative und quantitative Bestimmung der Anwesenheit des interessierenden Analyten dar.

Das erfindungsgemäße Verfahren kann für verschiedene Gruppen von Analyten eingesetzt werden. Nachstehend werden Beispiele derartiger Analytengruppen angegeben. Grundsätzlich ist dabei Voraussetzung, daß die Analyten eine identische Untereinheit oder einen kreuzreagierenden Bereich aufweisen.

Das vorgeschlagene Verfahren kann zur gleichzeitigen Bestimmung von Creatinkinease MB (CKMB) und Creaktinkinease BB (CKBB) eingesetzt werden. Hierbei wird als Detektor-Bindepartner ein spezifischer Bindepartner ausgewählt, der sowohl CKBB wie auch CKMB erkennt und als erster Fänger-Bindepartner wird ein Fänger-Bindepartner ausgewählt, der spezifisch für CKMB ist und nicht CKBB erkennt. Als zweiter Fänger-Bindepartner wird ein Fänger-Bindepartner ausgewählt, der spezifisch für CKBB ist und nicht CKMB erkennt. Die Anzahl der Analyten ist somit 2, die Anzahl der Detektor-Bindepartner 1 und die Anzahl der Fänger-Bindepartner 2. Bei der Bestimmung wird an einem ersten Oberflächenbereich die Konzentration von CKMB ermittelt und an einem zweiten Oberflächenbereich die Konzentration von CKBB.

Creatinkinease MB (CKMB) und Creaktinkinease BB (CKBB) können alternativ auch folgendermaßen bestimmt werden. Als Detektor-Bindepartner wird wie oben ein spezifischer Bindepartner ausgewählt, der sowohl CKBB wie auch CKMB erkennt. Als erster Fänger-Bindepartner wird ebenfalls ein Fänger-Bindepartner ausgewählt, der spezifisch für CKMB ist, und CKBB nicht erkennt. Als zweiter Fänger-Bindepartner wird jedoch ein spezifischer Fänger-Bindepartner ausgewählt, der sowohl CKBB als auch CKMB erkennt. Dies ist ein Fänger-Bindepartner, der spezifisch für die Untereinheit B von CKBB und CKMB ist. Die Anzahl der Analyten ist auch in dieser Variante 2, die Anzahl der Detektor-Bindepartner 1 und die Anzahl der Fänger-Bindepartner 2. Bei der Bestimmung wird an einem ersten Oberflächenbereich die Konzentration von CKMB ermittelt und an einem zweiten Oberflächenbereich die Summe der Konzentration von CKMB und CKBB.

Das vorgeschlagene Verfahren kann weiterhin zur gleichzeitigen Bestimmung von zumindest zwei Analyten in einer Probe eingesetzt werden, die aus Luteinisierendem Hormon (LH), Follikel-stimulierendem Hormon (FSH), Thyreoideastimulierendem Hormon (TSH) und humanem Choriongonadotropin (hCG) ausgewählt sind. Als Detektor-Bindepartner wird ein Bindepartner gewählt, der spezifisch die alpha-Untereinheit ist, die in allen Analyten dieser Gruppe identisch ist. Als Fänger-Bindepartner werden Bindepartner gewählt, die spezifisch für die jeweilige alpha-Untereinheit des zu bestimmenden Analyten sind.

Das vorgeschlagene Verfahren kann weiterhin zur gleichzeitigen Bestimmung von Protein-Isoformen eingesetzt werden. Werden als Analyten beispielsweise nicked hCG und non-nicked hCG verwendet, wird als Detektor-Bindepartner ein Bindepartner gewählt, der beide Isoformen erkennt, z.B. ein Detektor-Bindepartner der die alpha-Untereinheit erkennt. Als erster spezifischer Fänger-Bindepartner wird ein Bindepartner gewählt, der nur non-nicked hCG erkennt, und als zweiter spezifischer Fänger-Bindepartner ein Bindepartner gewählt, der nur nicked hCG erkennt. Die Anzahl der Analyten ist somit 2, die Anzahl der Detektor-Bindepartner 1 und die Anzahl der Fänger-Bindepartner 2.

Das vorgeschlagene Verfahren kann überdies zur gleichzeitigen Bestimmung von Immunoglobulin E-Antikörpern (Gesamt-IgE) und der Allergen-spezifischen IgE-Antikörper verwendet werden. Eine derartige Bestimmung von IgE ist insbesondere bei der Allergie-Diagnostik erforderlich. Bei der Bestimmung dieser Parameter d. h. von Gesamt-IgE und der Allergen-spezifischen IgE-Antikörper, wird als Detektor-Bindepartner ein Antikörper eingesetzt, der spezifisch für IgE ist. Beispielsweise kann bei der Bestimmung von humanem IgE als Detektor-Bindepartner der Antikörper IgE 60-4-4 (kommerziell erhältlich von pe-Diagnostik) eingesetzt werden. Als Fänger-Bindepartner für die Bestimmung des Parameters Gesamt-IgE wird in einem Oberflächenbereich wiederum der oben erwähnte Antikörper IgE 60-4-4 öder alternativ IgE 27-1-4 (kommerziell erhältlich von pe-Diagnostik) immobilisiert. Als weitere Fänger-Bindepartner für die Bestimmung der Spezifität der in der Probe enthaltenen IgE-Antikörper werden auf weiteren Oberflächenbereichen unterschiedliche Allergene immobilisiert. Bei der Bestimmung der Probe wird der Detektor-Bindepartner zunächst einige Minuten mit dieser Probe, welche die Analyten, d. h. die interessierenden, unterschiedlich spezifischen IgE-Antikörper enthält, inkubiert. Hierdurch bindet der Detektor-Bindungspartner an die unterschiedlichen Analyten, wodurch sich unterschiedliche Detektor-Analyt-Komplexe bilden. Anschließend wird diese Lösung über eine Oberfläche transportiert, auf der an unterschiedlichen Oberflächenbereichen die verschiedenen Fänger-Bindepartner immobilisiert sind. Der zeitliche Verlauf des Anstiegs der Fluoreszenz, welche durch das Anbinden der Detektor-Analyt-Komplexe an die Fänger hervorgerufen wird, mit einem Messgerät bestimmt.

Mit dem erfindungsgemäßen Verfahren können weiterhin freies Prostata-spezifisches Antigen (fPSA) und mit alpha-Antichymotrypsin (ACT) komplexiertes Prostata-spezifisches Antigen (PSA-ACT) gleichzeitig in einer Probe bestimmt werden. Die Probe kann weiterhin humanes Kallikrein 2 (hK2) als Analyten enthaltenen.

Ein Verfahren zum quantitativen Nachweis von fPSA, PSA-ACT und hK2, umfaßt dann folgende Schritte:

(a) Versetzen einer Probe, die fPSA, PSA-ACT und hK2 enthält, mit einem markierten Detektor-Bindepartner unter Bildung von Detektor-Analyt-Komplexen, die aus jeweils einem Analyt- und einem Detektormolekül bestehen;

(b) Anbinden der in Schritt (a) erhaltenen Detektor-Analyt-Komplexe an drei Fänger-Bindepartner unter Bildung von Detektor-Analyt-Fänger-Komplexen, wobei jeder Fänger-Bindepartner für einen Detektor-Analyten-Komplex spezifisch ist; und

(c) zeitabhängige Erfassung der Bildung der Detektor-Analyt-Fänger-Komplexe.

Ein alternatives Verfahren zum quantitativen Nachweis von fPSA, PSA-ACT und hK2 umfaßt folgende Schritte:

(a) Versetzen einer Probe, die fPSA, PSA-ACT und hK2 enthält, mit einem markierten Detektor-Bindepartner unter Bildung von Detektor-Analyt-Komplexen, die aus jeweils einem Analyt- und einem Detektormolekül bestehen;

(b) Anbinden der in Schritt (a) erhaltenen Detektor-Analyt-Komplexe an drei Fänger-Bindepartner unter Bildung von Detektor-Analyt-Fänger-Komplexen, wobei einer der Fänger-Bindepartner für Detektor-fPSA-Komplexe spezifisch ist, ein weiterer der Fänger-Bindepartner für Detektor-hK2-Komplexe spezifisch ist und an einen dritten der Fänger-Bindepartner sowohl Detektor-fPSA-Komplexe als auch Detektor-PSA-ACT-Komplexe anbinden können; und

(c) zeitabhängige Erfassung der Bildung der Detektor-Analyt-Fänger-Komplexe.

Sollen lediglich fPSA und PSA-ACT bestimmt werden, umfaßt ein Verfahren zur Bestimmung dieser Analyten die Schritte:

(a) Versetzen einer Probe, die fPSA und PSA-ACT enthält, mit einem markierten Detektor-Bindepartner unter Bildung von Detektor-Analyt-Komplexen, die aus jeweils einem Analyt- und einem Detektormolekül bestehen;
(b) Anbinden der in Schritt (a) erhaltenen Detektor-Analyt-Komplexe an zwei Fänger-Bindepartner unter Bildung von Detektor-Analyt-Fänger-Komplexen, wobei einer der Fänger-Bindepartner für Detektor-fPSA-Komplexe spezifisch ist und an einen der Fänger-Bindepartner Detektor-fPSA-Komplexe und Detektor-PSA-ACT-Komplexe anbinden können; und
(c) zeitabhängige Erfassung der Bildung der Detektor-Analyt-Fänger-Komplexe.

hK2, das gegebenenfalls zusätzlich zu fPSA und PSA-ACT in der Probe enthalten ist, stört bei der Bestimmung von fPSA und PSA-ACT durch dieses Verfahren nicht.

Nachfolgend werden Ausführungsformen der Erfindung anhand der Zeichnungen näher erläutert. Es zeigen:

Fig. 1 den Schritt (a) eines ersten Verfahrens zur Bestimmung von fPSA, PSA-ACT und hK2;

Fig. 2a den Schritt (b) des ersten Verfahrens zur Bestimmung von fPSA, PSA-ACT und hK2;

Fig. 2b einen modifizierten Schritt (b) des ersten Verfahrens zur Bestimmung von fPSA, PSA-ACT und hK2;

Fig. 3 den Schritt (a) eines zweiten Verfahrens zur Bestimmung von fPSA, PSA-ACT und hK2;

Fig. 4 den Schritt (b) des zweiten Verfahrens zur Bestimmung von fPSA, PSA-ACT und hK2;

Fig. 5 eine Meßkurve zur Aufnahme der Kalibrierfunktionen (1) und (2) bei der Bestimmung von fPSA und tPSA;

Fig. 6 eine Meßkurve zur Aufnahme der Kalibrierfunktion (3) bei der Bestimmung von fPSA und tPSA;

Fig. 7 eine graphische Darstellung der Anbindungsgeschwindigkeit von Detektor-fPSA- und Detektor-PSA-ACT-Komplexen an Fänger-Bindepartner;

Fig. 8 eine Meßfunktion zur Bestimmung von fPSA und tPSA in einer unbekannten Probe und

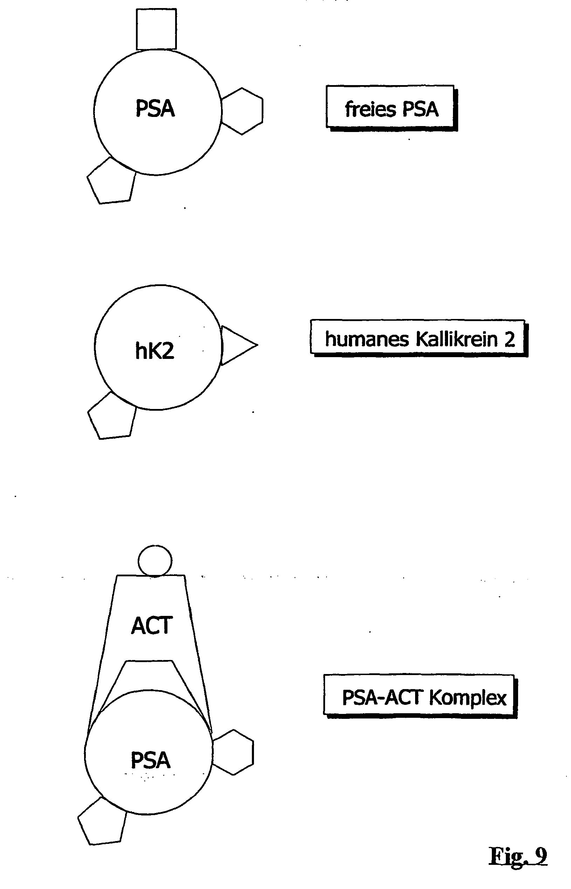

Fig. 9 eine Legende für die in den Figuren 1 bis 4 verwendeten Symbole.

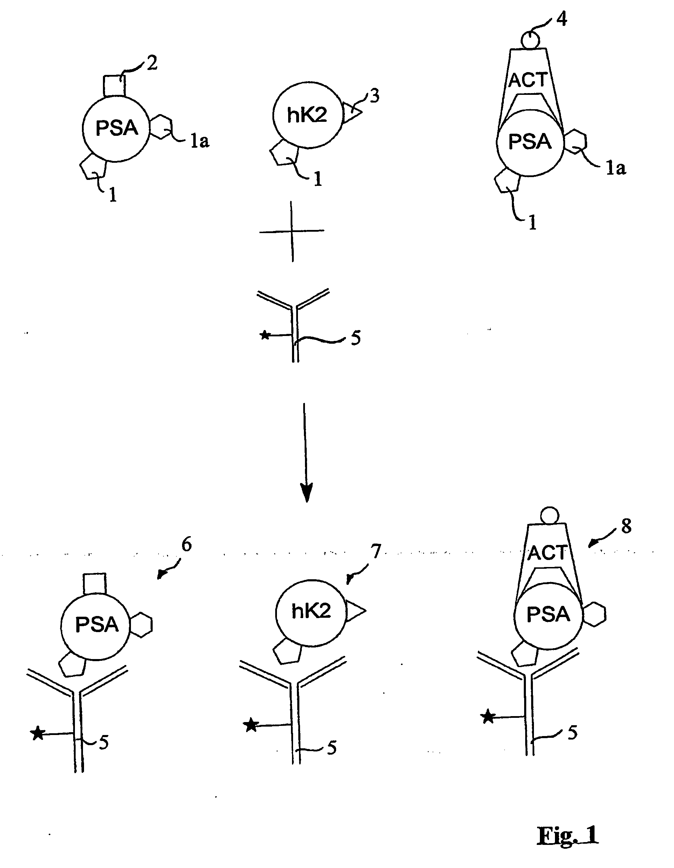

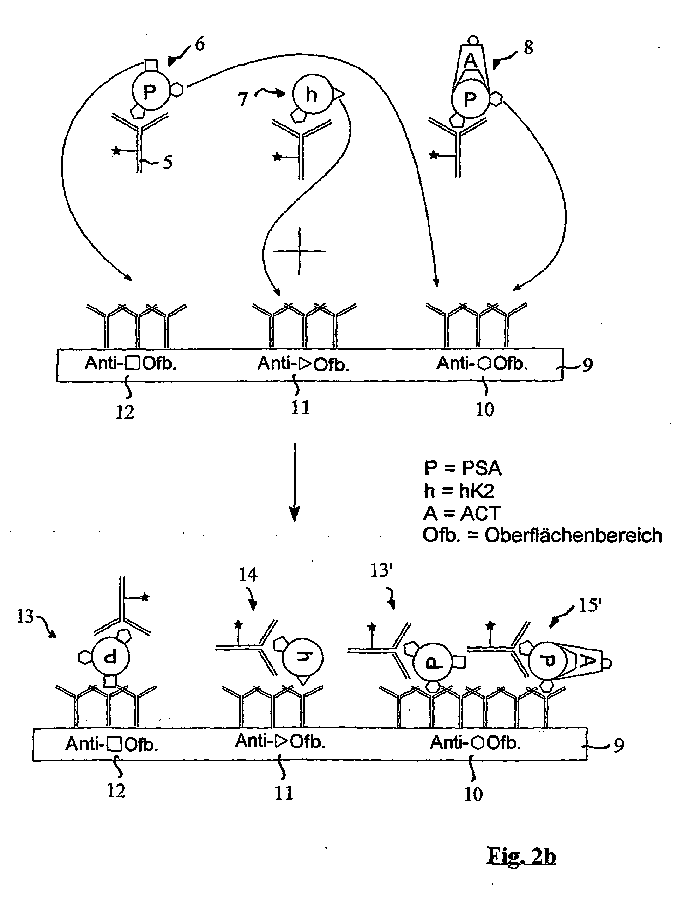

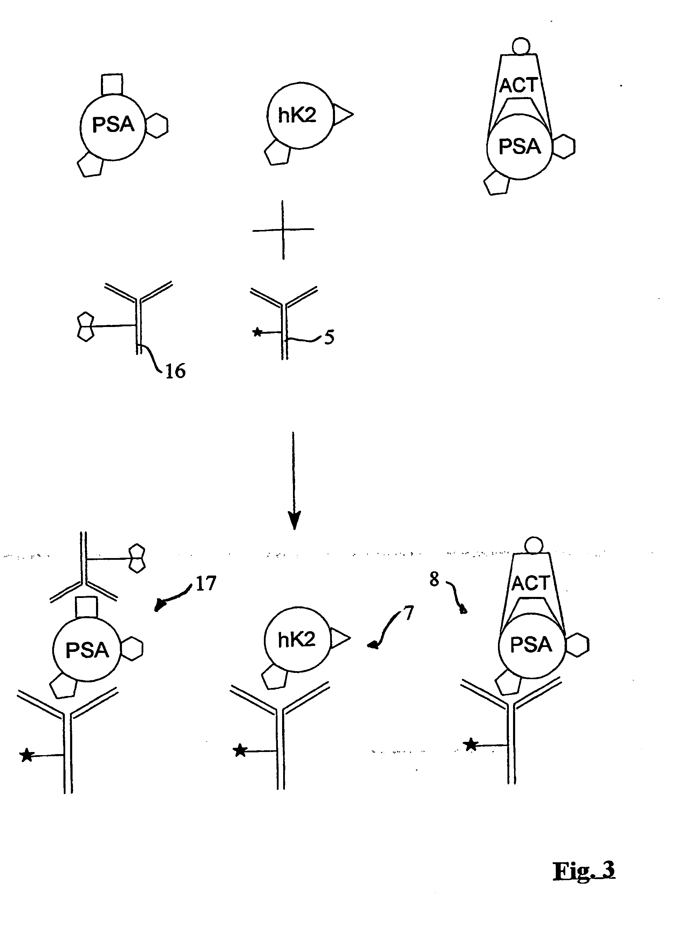

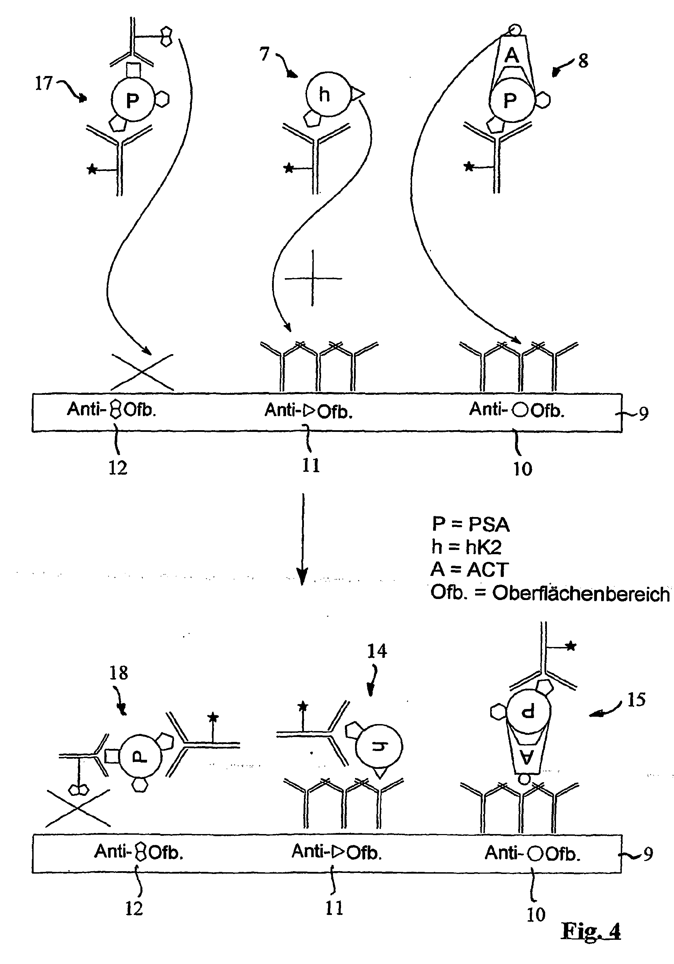

In den Figuren 1 bis 4 wird die Verwendung des erfindungsgemäßen Verfahrens zur quantitativen Bestimmung des prostataspezifischen Antigens (PSA) gezeigt. Dabei werden freies PSA (fPSA), mit alpha-Antichymotrypsin komplexiertes PSA (PSA-ACT) sowie humanem Kallikrein-2 (hk2) bestimmt (Fig. 1, Fig. 3).

Als Detektor-Bindepartner wird nach Fig. 1 ein markierter Bindepartner 5 ausgewählt, der eine Bindungsstelle 1 erkennt, die auf allen drei Analyten vorkommt. Ein für diesen Zweck geeigneter Detektor-Bindepartner 5 ist der Antikörper PS 2 (Hytest), der das PSA-Epitop 3 erkennt und auch 100%ig mit hk2 kreuzregiert. Es können jedoch auch andere Antikörper eingesetzt werden, solange sie die genannten Bedingungen für den Detektor-Bindepartner 5 erfüllen.

Der als Detektor-Bindepartner 5 verwendete Antikörper ist mit einem Fluoreszenzfarbstoff (*) markiert. Dazu können 0,001 bis 10 (vorzugsweise 2 bis 5) Moleküle eines aktivierten Fluoreszenzfarbstoffes unter Verwendung eines Standardverfahrens an diesen Antikörper gekoppelt werden. Für diesen Zweck geeignete Fluoreszenzfarbstoffe sind beispielsweise S0458 (FEW, Wolfen) oder die Cyanfarbstoffe Cy5 (Amersham).

Die nachzuweisenden Analyten (fPSA, hk2 und PSA-ACT) werden einige Minuten mit der Probe, die diese Analyten enthält, unter Bildung von Detektor-Analyt-Komplexen 6, 7, 8 inkubiert. Die Inkubationszeit ist von der Temperatur sowie der Konzentration des Detektor-Bindepartners 5 abhängig. Wird als Antikörper PS2 verwendet, so beträgt die Inkubationszeit bei einer Temperatur von 37 °C und einer Konzentration des Detektor-Bindepartners 5 von 0,1 bis 1000 μg/ml 1 bis 120 Minuten, bei einer bevorzugten Konzentration des Detektor-Bindepartners 5 von 5 μg/ml vorzugsweise 10 Minuten.

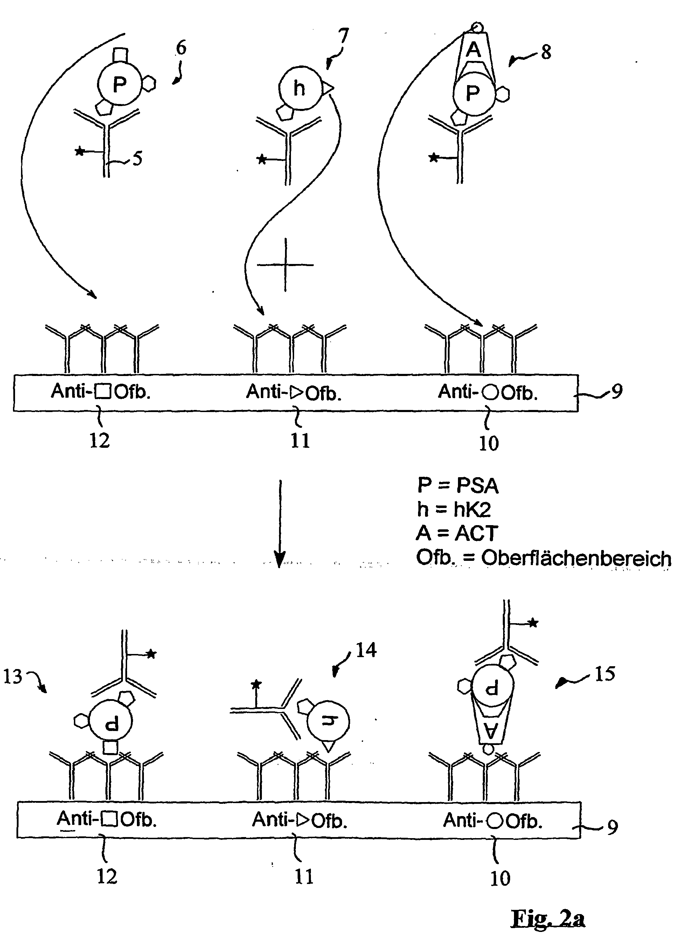

Anschließend wird die so erhaltene Lösung aus Detektor-Analyt-Komplexen 6, 7, 8 mit einer Oberfläche 9 in Kontakt gebracht, die gesonderte Oberflächenbereiche 10, 11, 12, auf denen verschiedene Fänger-Bindepartner immobilisiert sind, aufweist (Fig. 2a).

Auf einem ersten Oberflächebereich 10 dieser Oberfläche 9 ist ein Fänger-Bindepartner immobilisiert, der spezifisch ACT oder einen Epitop 4 im PSA-ACT-Komplex 8 erkennt, der nicht im freien PSA vorkommt, und ebenfalls in der Lage ist, mit dem zuvor gewählten Detektor-Bindepartner 5 und PCT-ACT ein Sandwich 15 zu bilden. Hierfür ist beispielsweise ein polyklonaler Antikörper gegen ACT, wie beispielsweise die polyklonalen Antikörper Rabbit-anti-ACT oder Sheep-anti-ACT (DPC Biermann) geeignet. Alternativ kann aber auch ein monoklonaler Antikörper gegen ACT eingesetzt werden, wie beispielsweise der Clon 8e6, 22h9/33 oder aber auch ACT 14c7 (DPC Biermann).

Auf einem zweiten Oberflächenbereich 11 ist ein Fänger-Bindepartner immobilisiert, der ein Epitop 3 erkennt, welches spezifisch für hk2 ist und ebenfalls in der Lage ist, mit dem eingesetzten Detektor-Bindepartner 5 und hK2 ein Sandwich 14 zu bilden (Fig. 2a, Mitte). Hierfür können Antikörper verwendetet werden, welche nach den von Tindall et all in US 5,526,639 bzw. WO 95/0334 beschriebenen Verfahren hergestellt wurden.

Auf einem dritten Oberflächenbereich 12 ist ein Fänger-Bindepartner immobilisiert, der ein Epitop 2 erkennt, das ausschließlich auf fPSA vorkommt, und in der Lage ist, mit dem zuvor gewählten Detektor-Bindepartner 5 und fPSA ein Sandwich 13 zu bilden (Fig. 2a, links außen). Zu diesem Zweck kann der Antikörper PS1 (Hytest) verwendet werden. Andere Antikörper, die ebenfalls spezifisch für fPSA sind und in der Lage sind, mit dem zuvor gewählten Detektor-Bindepartner 5 ein Sandwich zu bilden, wie beispielsweise der Antikörper 8a6 (Hytest), sind dazu ebenfalls geeignet.

Alternativ kann die Oberfläche zur Bestimmung der oben genannten Analyten auch anders gestaltete Oberflächenbereiche aufweisen. Eine derartige Alternative ist in Fig. 2b dargestellt.

Auf dem ersten Oberflächenbereich 10 (Fig. 2b, rechts außen) dieser Variante ist ein Fänger-Bindepartner immobilisiert, der einen Epitop 1a erkennt, der sowohl in fPSA als auch im PSA-ACT-Komplex vorkommt und ebenfalls in der Lage ist, mit dem zuvor gewählten Detektor-Bindepartner 5 und fPSA und PSA-ACT Sandwich 13' und Sandwich 15' zu bilden. Zu diesem Zweck kann der Antikörper 5g6 (Hytest) verwendet werden. Auf diesem Oberflächenbereich 10 wird somit Gesamt-PSA (tPSA), d. h. die Summe aus freiem PSA und komplexiertem PSA bestimmt .

Der zweite und dritte Oberflächenbereich 11, 12 entsprechen in dieser Variante den obigen Oberflächenbereichen. Da für die Quantifizierung die Anbindegeschwindicrkeit der Detektor-Analyt-Komplexe an die Fänger-Bindepartner entscheidend ist, wird somit auf dem ersten Oberflächenbereich 10 tPSA bestimmt werden und auf dem dritten Oberflächenbereich 12 fPSA. Aus der Differenz kann dann die Menge an komplexiertem PSA (PSA-ACT) errechnet werden.

Der zeitliche Verlauf der Anbindung der Detektor-Analyt-Komplexe an die Fänger-Bindepartner auf den verschiedenen Oberflächenbereichen wird bestimmt. Dazu können die in DE 19628002 und DE 19711281 beschriebenen Vorrichtungen verwendet werden. Auf diese Weise wird ein Wert für die Anbindungsgeschwindigkeit erhalten, der als mV/s angegeben werden kann. Typische Werte liegen in Abhängigkeit von der Analytenkonzentration in der Probe zwischen 10 und 10.000 mV/s.

Als Detektor-Bindepartner 5 wird nach Fig. 3 ebenso wie in Fig. 1 ein markierter Bindepartner ausgewählt, der eine Bindungsstelle 1 erkennt, die auf allen drei Analyten vorkommt. Die Probe wird jedoch zusätzlich zu dem Detektor-Bindepartner 5 mit einem dritten Bindepartner 16 versetzt, der spezifisch für fPSA ist. An den Analyt fPSA binden somit zwei Bindepartner während der Inkubation an. Im Gegensatz zu den in Fig. 2a und 2b dargestellten Anbindungsschritten an die immbolisierten Fänger-Bindepartner wird in die Bindung zwischen dem Detektor-fPSA-Komplex 17 und dem Fänger-Bindepartner unter Bildung von Komplex 18 nicht über das fPSA-Molekül, sondern über den dritten Bindepartner 16 hergestellt.

Beispiel

In diesem Beispiel wird die Bestimmung von fPSA und tPSA und PSA-ACT durch das erfindungsgemäße Verfahren beschrieben.

1. Verwendete Bindepartner

Als Detektor-Bindepartner wird der Antikörper Anti-PSA-Klon PS2 (Hytest) verwendet, der das PSA-Epitop 3 erkennt und deshalb in der Lage ist, sowohl an fPSA als auch an PSA-ACT-Komplexe anzubinden. Dieser Antikörper wird im folgenden als Detektor-Antikörper bezeichnet.

Als Fänger-Bindepartner werden die Antikörper Anti-PSA-Klon PS1 (Hytest) und Anti-PSA-Klon 5g6 (Hytest) verwendet . Anti-PSA-Klon PS1 ist ein Antikörper der ausschließlich fPSA erkennt. Anti-PSA-Klon 5g6 ist ein Antikörper, der ein Epitop erkennt, das sowohl in fPSA und PSA-ACT vorhanden ist. Diese Antikörper werden im folgenden als Fänger-Antikörper bezeichnet.

2. Markierung des Detektor-Antikörpers

1 mg des Antikörpers Anti-PSA Klon PS2 (Hytest) mit einer Konzentration von 1 mg/ml wurde mit 95 $\mu$g des Fluoreszenzfarbstoffs S 0458 (FEW Wolfen), gelöst in 20 $\mu$l DMSO (Sigma), versetzt und 20 min bei Raumtemperatur gerührt. Das Reaktionsgemisch wird anschließend über eine FPLC (Äkta purifier; Amersham pharmacia biotech) mit Hilfe einer Säule (Hi Trap Desalting, Fa: Amersham pharmacia biotech) gereinigt. An jeden der erhaltenen Antikörper waren etwa 0,01-15 Farbstoffmoleküle (genauer 2-6 Farbstoffmoleküle) gekoppelt.

### 3. Immobilisierung der Fänger-Antikörper

Auf einem Prisma aus Polymethylmethacrylat (PMMA, kommerziell erhältlich von Leica) wird durch Dispensieren einer Linie bestehend aus 11 ineinander verlaufenden Tropfen, jeder mit einem Volumen von 20 nl, einer Lösung des Anti-PSA-Antikörpers Klon PS1 (c = 0,5 mg/ml) in PBS-Puffer (pH 7,4, mit 500 mmol Natriumchlorid) der erste Oberflächenbereich erzeugt. Desgleichen wird parallel zu diesem Oberflächenbereich durch Dispensieren einer weiteren Linie, bestehend aus 11 ineinander verlaufenden Tropfen, jeder mit einem Volumen von 20 nl, einer Lösung des Anti-PSA Antikörper Klon 5g6 (c = 0,5 mg/ml) in PBS-Puffer, der zweite Oberflächenbereich erzeugt.

Nach dem Eintrocknen der Linien wird das Prisma in eine Vorrichtung gegeben, die in DE 196 28 002 beschrieben wird. Die immobilisierten Fänger-Antikörper befinden sich dann im Fließkanal des Sensors.

### 4. Aufnahme einer Kalibrierfunktion

Die Kalibrierung wurde mit Hilfe bekannter Analyt-Konzentrationen vorgenommen. Zu diesem Zweck wurden kommerziell erhältliches fPSA (Quartett, 7820-0604) und PSA-ACT-Komplex (DPC Biermann BA 1022) verwendet. Diese Analyten werden im folgenden als Antigene bezeichnet.

Für die Aufnahme der Kalibrierfunktion wurde eine Mischung aus 50 $\mu$l des markierten Detektor-Antikörpers Anti-PSA-Klon PS2 in einer Konzentration von 6 $\mu$g/ml mit je 50 $\mu$l der verschiedenen bekannten Konzentrationen der Antigene (fPSA bzw. PSA-ACT-Komplex) in PBS-Puffer (mit 1% BSA) zusammengegeben und für 10 min bei 35 °C inkubiert. Die für die Kalibration eingesetzten Konzentrationen sind in Tabelle 1 widergegeben.

Anschließend werden je Konzentration und je Antigen 100 $\mu$l der Kalibrierlösung in den Biosensor gegeben und die Zunahme der Fluoreszenz, hervorgerufen durch die Anbindung der Detektor-Antigen-Komplexe an die jeweiligen spezifischen Oberflächenbereiche, bei einer Photomultiplerspannung von 700 V gemessen. Das Meßprinzip ist aus Meusel et al, Anal. Chem. 1999, 71, 5430-5435, DE 19628002 und DE 19711281 bekannt. Als Meßgerät kann die in WO 01/77645A1 beschriebene Vorrichtung verwendet werden.

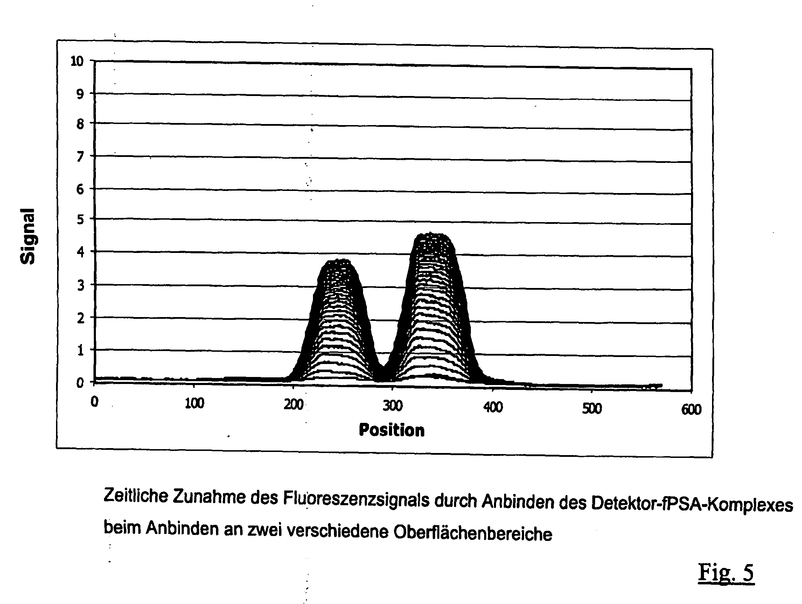

Bei der Kalibrierung mit fPSA erfolgt eine Anbindung des Detektor-fPSA-Komplexes an beide Oberflächenbereiche, da beide Oberflächenbereiche, sowohl der erste Oberflächenbereich (auf dem der Anti-PSA Antikörper PS1 immobilisiert ist) als auch der zweite Oberflächenbereich (auf dem der Anti-PSA Antikörper 5g6 immobilisiert ist) fPSA binden können. Fig. 5 zeigt die zeitliche Zunahme des Fluoreszenzsignals durch Anbinden des Detektor-fPSA-Komplexes an den ersten Oberflächenbereich (Position 200-300) und den zweiten Oberflächenbereich (Position 300-400). Die in Fig. 5 dargestellte Meßkurve dient zur Aufnahme der Kalibrierfunktionen (1) und (2) (siehe unten).

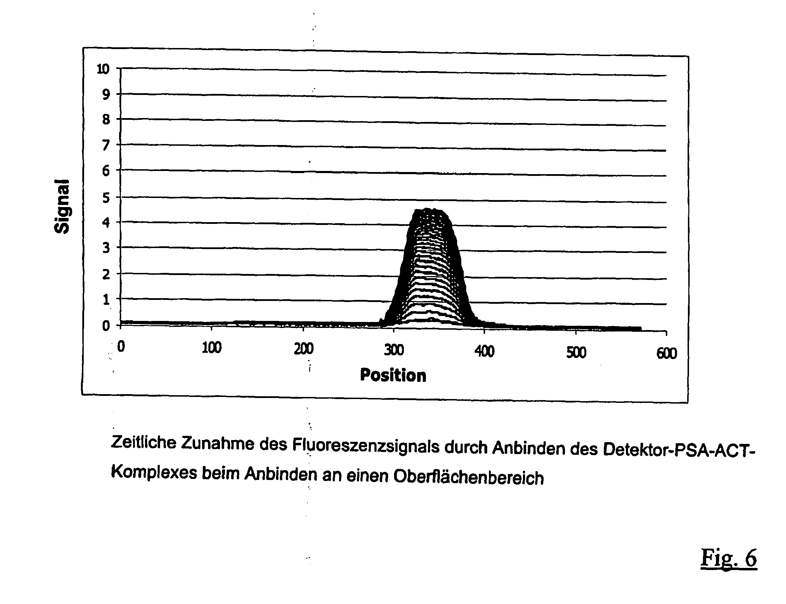

Bei der Kalibrierung mit PSA-ACT erfolgt nur eine Anbindung des Detektor-PSA-ACT-Komplexes an den zweiten Oberflächenbereich (Anti-PSA-Antikörper 5g6). Nur dieser Oberflächenbereich erkennt neben Detektor-fPSA-Komplex auch Detektor-PSA-ACT Komplex. Fig. 6 zeigt die zeitliche Zunahme des Fluoreszenzsignals durch Anbindung des Detektor-PSA-ACT-Komplexes im zweiten Oberflächenbereich (Position 300-400). Im ersten Oberflächenbereich (Position 200-300) erfolgt keine Anbindung des Detektor-PSA-ACT-Komplexes, da dieser Bereich spezifisch für den Detektor-fPSA-Komplex ist. Die in Fig. 6 dargestellte Meßkurve dient zur Aufnahme der Kalibrierfunktion (3) (siehe unten).

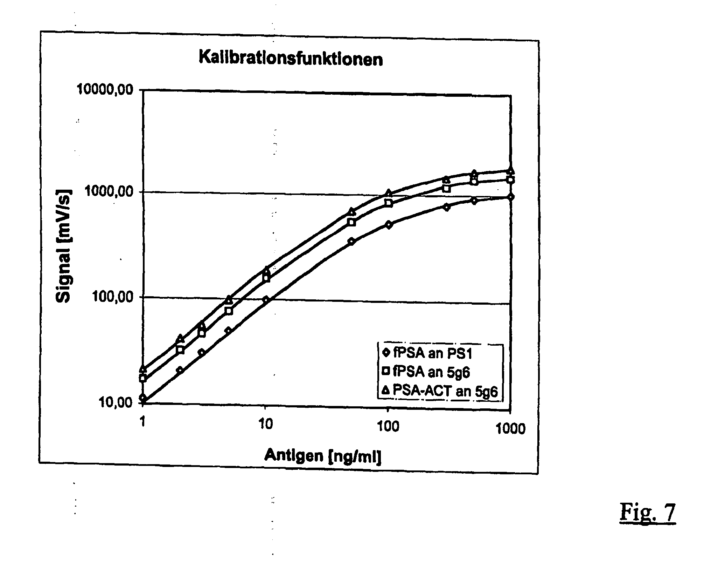

Aus diesen Daten konnte spezifisch für jeden der beiden Oberflächenbereiche getrennt die könzentrationsabhängigen Anbindungsgeschwindigkeiten der unterschiedlichen Detektor-Antigen-Komplexe bestimmt werden. Die konzentrationsabhängige Anbindungsgeschwindigkeit wird in der Zunahme des Signals widergespiegelt. Der Signalanstieg ist also von Bedeutung, nicht die absolute Höhe. In Tabelle 1 und in Fig. 7 sind die ermittelten Daten graphisch dargestellt.

Tabelle 1

| Konzentration an fPSA bzw. PSA-ACT* [ng/ml] | Anbindung von fPSA an PS1 [mV/s] | Anbindung von fPSA an 5g6 [mV/s] | Anbindung von PSA-ACT an PS1 [mV/s] | Anbindung von PSA-ACT an 5g6 [mV/s] |
|---|---|---|---|---|
| 1 | 11,39 | 17,45 | 0 | 21,71 |
| 2 | 21,10 | 33,53 | 0 | 43,18 |
| 3 | 31,87 | 48,55 | 0 | 58,80 |
| 5 | 51,88 | 78,14 | 0 | 98,63 |
| 10 | 99,38 | 156,1 | 0 | 185,9 |
| 50 | 360,5 | 548,9 | 0 | 696,5 |

(fortgesetzt)

| Konzentration an fPSA bzw. PSA-ACT* [ng/ml] | Anbindung von fPSA an PS1 [mV/s] | Anbindung von fPSA an 5g6 [mV/s] | Anbindung von PSA-ACT an PS1 [mV/s] | Anbindung von PSA-ACT an 5g6 [mV/s] |
|---|---|---|---|---|
| 100 | 526,5 | 838,3 | 0 | 1058 |
| 300 | 788,1 | 1191 | 0 | 1481 |
| 500 | 923,1 | 1406 | 0 | 1717 |
| 1000 | 1004 | 1494 | 0 | 1859 |
| * Die Konzentration ist jeweils auf das immunochemisch nachweisbare PSA bezogen. | | | | |

Es wurden die folgenden drei Kalibrationsfunktionen der Form

$$c_x = \frac{A_i * S_{x-y}}{B_i - S_{x-y}} \ .$$

gefunden. Die verwendeten Symbole haben folgende Bedeutung:

$c_x$= Konzentration des Analyten x in der Lösung,
$S_{x-y}$ = Anbindungsgeschwindigkeit des Analyten x an den Oberflächenbereich y und
B, A = Parameter der Kalibrationsfunktionen;
x= Index für den Analyten
y= Index für den Oberflächenbereich
i = Index für die Kalibrierfunktion.

$$(1) \qquad c_{fPSA} = \frac{A_1 * S_{fPSA-PS1}}{B_1 - S_{fPSA-PS1}}$$

mit A1 = 109,000 ng/ml und B1 = 1107,93 mV/s;

$$(2) \qquad c_{fPSA} = \frac{A_2 * S_{fPSA-5G6}}{B_2 - S_{fPSA-5G6}}$$

mit A2 = 99,6353 ng/ml und B2 = 1645,45 mV/s;

$$(3) \qquad c_{PSA-ACT} = \frac{A_3 * S_{PSA-ACT-5G6}}{B_3 - S_{PSA-ACT-5G6}}$$

mit A3 = 95,7948 ng/ml und B3 = 2023,62 mV/s.
Für die Berechnung der Konzentrationen wurden folgende Formeln verwendetet, welche die Parameter der Kalibrationsfunktionen verwenden.

$$(4) \qquad S_{fPSA-PS1} = \frac{c_{fPSA} * B_1}{A_1 + c_{fPSA}}$$

$$(5) \qquad S_{tPSA-5G6} = \frac{C_{fPSA} * B_2}{A_2 + c_{fPSA}} + \frac{c_{PSA-ACT} * B_3}{A_3 + C_{PSA-ACT}}$$

$$(6) \qquad C_{fPSA} = \frac{A_1 * S_{fPS1-PS1}}{B_1 - S_{fPS1-PS1}}$$

$$(7) \qquad S_{fPSA-5G6} = \frac{c_{fPSA} * B_2}{A_2 + c_{fPSA}}$$

$$(8) \qquad C_{PSA-ACT} = \frac{S_{fPSA-5G6} - S_{PSA-ACT-5G6} * A_3}{S_{PSA-ACT-5G6} - B_3}$$

5. Bestimmung von fPSA und tPSA in einer unbekannten Probe

Bei der Bestimmung der unbekannten Probenlösung wurde analog zur Aufnahme der Kalibrierfunktion vorgegangen, außer daß statt einer bekannten Antigen-Konzentration die Probe mit einer unbekannten Konzentrationen dieser Antigene eingesetzt wurde.

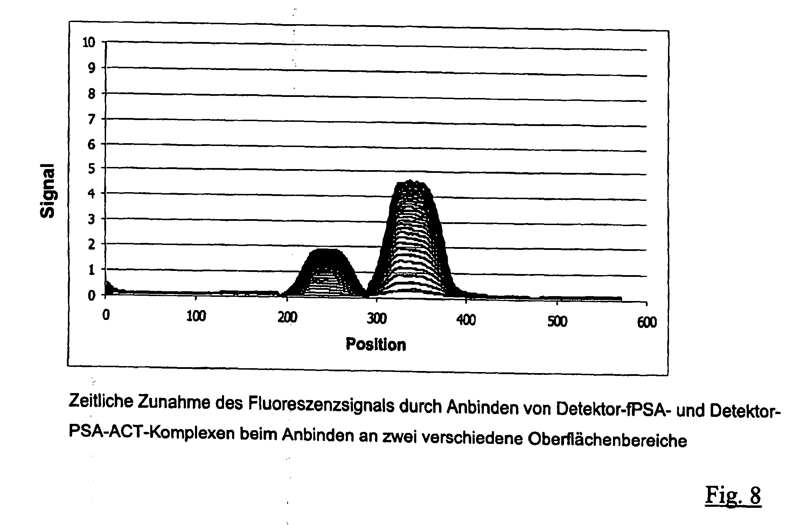

50 $\mu$l der unbekannten Probe werden mit 50 $\mu$l des markierten Detektor-Antikörpers Anti-PSA Klon PS2 in einer Konzentration von 6 $\mu$g/ml zusammengegeben und für 10 min bei 35°C inkubiert. Anschließend wird die Lösung in den Biosensor gegeben und die Zunahme der Fluoreszenz, hervorgerufen durch die Anbindung der Detektor-Antigen-Komplexe an die jeweiligen spezifischen Oberflächenbereiche bei einer Photomultiplierspannung von 700 V gemessen. Die Meßfunktion ist in Fig. 8 dargestellt. Daraus ergeben sich für den ersten Oberflächenbereich (Position 200-300), auf dem PS 1 immobilisiert ist, eine Anbindungsgeschwindigkeit von 14,8 mV/s und für den zweiten Oberflächenbereich (Position 300-400), auf dem 5g6 immobilisiert ist, eine Anbindungsgeschwindigkeit von 540 mV/s.

Zur Auswertung dieser Meßfunktion wird die Tatsache genutzt, daß sowohl fPSA als auch PSA-ACT unabhängig voneinander am zweiten Oberflächenbereich anbinden, während im ersten Oberflächenbereich ausschließlich fPSA anbindet. Zunächst wurde aus der Anbindungsgeschwindigkeit im ersten Oberflächenbereich die Konzentration des fPSA in der Lösung bestimmt. Die Anstiegsgeschwindigkeit (y) von 14,8 mV/s entspricht einer Konzentration von 1,5 ng/ml fPSA (siehe Gleichung 6, unten). Da fPSA jedoch nicht nur am ersten Oberflächenbereich, sondern gleichzeitig auch am zweiten Oberflächenbereich anbindet, muß dies für die Bestimmung von PST-ACT berücksichtigt werden, das ebenfalls am zweiten Oberflächenbereich anbindet, d. h. der dort bestimmte Anstieg muß um den Betrag bereinigt werden, der auf fPSA zurückzuführen ist.

Die 1,5 ng/ml fPSA, die am ersten Oberflächenbereich angebunden haben, liefern zusätzlich am zweiten Oberflächenbereich einen Beitrag von 24,0 mV/s (siehe Gleichung 7, unten). D. h. ein Beitrag von 24,0 mV/s des Signals am zweiten Oberflächenbereich ist auf die Anbindung von fPSA zurückzuführen, während 516 mV/s (= 540 mV/s - 24,0 mV/s) auf das Anbinden von PSA-ACT zurückzuführen sind.

Diese 516 mV/s werden laut Kalibrierkurve für das Anbinden von PSA-ACT an dem zweiten Oberflächenbereich durch das Anbinden von PSA-ACT aus einer Konzentration von 34,8 ng/ml PSA-ACT hervorgerufen.

Alternativ zu der hier vorgestellten Methode, die die Bestimmung der Anbindung von fPSA an den ersten Oberflächenbereich, die Bestimmung der Anbindung von fPSA und PSA-ACT an den zweiten Oberflächenbereich und die Berechnung der Anbindung von PSA-ACT an den zweiten Oberflächenbereich umfaßt, kann auch mit nur einer Kalibrierlösung, welche eine Mischung von fPSA und PSA-ACT enthält, kalibriert werden. Hierbei wird die Anbindung von fPSA an den ersten Oberflächenbereich und die Anbindung der Mischung (fPSA und PSA-ACT) an den zweiten Oberflächenbereich bestimmt. Allerdings vergrößert sich der Fehler der Bestimmung von PSA-ACT, je weiter die zu bestimmende Probe von der Kalibriermischung abweicht.

**Bezugszeichenliste**

| | |
|---|---|
| 1 | Bindungsstelle, die auf fPSA, hK2 und PSA-ACT vorkommt |
| 1a | Bindungsstelle, die nur auf fPSA und PSA-ACT vorkommt |
| 2 | Bindungsstelle, die nur auf fPSA vorkommt |
| 3 | Bindungsstelle, die nur auf hK2 vorkommt |
| 4 | Bindungsstelle, die nur auf ACT vorkommt |
| 5 | markierter Detektor-Bindepartner (* = Markierung) |
| 6 | Detektor-fPSA-Komplex |
| 7 | Detektor-hK2-Komplex |
| 8 | Detektor-PSA-ACT-Komplex |
| 9 | Oberfläche |
| 10 | erster Oberflächenbereich |
| 11 | zweiter Oberflächenbereich |
| 12 | dritter Oberflächenbereich |
| 13, 13' | Detektor-fPSA-Fängerkomplex |
| 14 | Detektor-hK2-Fängerkomplex |
| 15, 15' | Detektor-PSA-ACT-Fängerkomplex |
| 16 | dritter Bindungspartner |
| 17 | Detektor-fPSA-Komplex mit drittem Bindungspartner |
| 18 | Detektor-fPSA-Fängerkomplex mit drittem Bindungspartner |

**Patentansprüche**

1. Verfahren zum quantitativen Nachweis von mehreren unterschiedlichen Analyten, wobei die Anzahl n der Analyten zumindest 2 beträgt, umfassend die Schritte

(a) Versetzen einer Probe, die die n Analyten enthält, mit zumindest einem markierten Detektor-Bindepartner unter Bildung von Detektor-Analyt-Komplexen, die aus jeweils einem Analyt- und mindestens einem Detektormolekül bestehen, wobei die Anzahl x der Detektor-Bindepartner kleiner oder gleich n - 1 ist, jeder der Detektor-Bindepartner an zumindest einen Analyten anbinden und zumindest einer der Detektor-Bindepartner an zumindest zwei Analyten anbinden kann;
(b) Anbinden der in Schritt (a) erhaltenen Detektor-Analyt-Komplexe an Fänger-Bindepartner unter Bildung von Detektor-Analyt-Fänger-Komplexen, wobei die Anzahl y der Fänger-Bindeparter der Anzahl n der Analyten entspricht und jeder Fänger-Biridepartner für zumindest einen Detektor-Analyten-Komplex spezifisch ist; und
(c) zeitabhängige Erfassung der Bildung der Detektor-Analyt-Fänger-Komplexe.

2. Verfahren nach Anspruch 1, wobei der Probe in Schritt (a) zumindest ein dritter Bindepartner zugesetzt wird, der spezifisch für einen Analyten ist und an einer Bindungsstelle des Analyten anbinden kann, die sich von der Bindungsstelle, an die die Detektor-Bindungspartner anbinden können, unterscheidet, wobei die so erhaltenen Detektor-Analyt-Komplexe, die den dritten Bindepartner umfassen, in Schritt (b) über den dritten Bindepartner an den immbolisierten, für den Analyten spezifischen Fänger-Bindepartner anbinden.

3. Verfahren nach einem der vorstehenden Ansprüche, wobei zumindest einer der Dektektor-Analyt-Komplexe an zwei verschiedene Fänger-Bindepartner anbinden kann.

4. Verfahren nach einem der vorstehenden Ansprüche, wobei der Detektor-Bindungspartner mit einem Fluoreszenz- oder Lumineszenzfarbstoff markiert ist.

5. Verfahren nach einem der vorstehenden Ansprüche, wobei die Detektor-Bindepartner, Fänger-Bindepartner und dritten Bindepartner Liganden, polyklonale und monoklonale Antikörper und deren Antigen bindende Fragmente; RNA; DNA; DNA/RNA-Derivate und deren Analoga, wie beispielsweise Aptamere; Proteine, wie beispielsweise Lectine; und Allergene umfassen.

6. Verfahren nach einem der vorstehenden Ansprüche, wobei die Fänger-Bindepartner auf unterschiedlichen Oberflächenbereichen einer Oberfläche immobilisiert sind.

7. Verfahren nach Anspruch 6, wobei die Fänger-Bindepartner an die Oberfläche direkt über hydrophobe/ionische Wechselwirkungen oder durch chemische Bindung gebunden werden.

8. Verfahren nach Anspruch 6, wobei die Fänger-Bindepartner an die Oberfläche indirekt über einen auf der Oberfläche befindlichen Immobilisierungsvermitteler, wie einem Anti-Antikörper, Avidin oder ein Carrierprotein, gebunden werden.

9. Verfahren nach einem der vorstehenden Ansprüche, wobei die zeitabhängige Erfassung der Bildung der Detektor-Analyt-Fänger-Komplexe durch Messungen an zumindest zwei unterschiedlichen Zeitpunkten erfolgt.

10. Verfahren nach einem der Ansprüche 1 bis 8, wobei die zeitabhängige Erfassung der Bildung der Detektor-Analyt-Fänger-Komplexe am Endpunkt der Bildung der Detektor-Analyt-Fänger-Komplexe erfolgt.

11. Verfahren nach einem der Ansprüche 2 bis 10, wobei die Konzentration der Detektor-Analyt-Fänger-Komplexe durch Bestimmung der zeitabhängigen Zunahme an Fluoreszenzlicht erfaßt wird.

12. Verfahren nach einem der vorstehenden Ansprüche, wobei die aus der Probe zu bestimmenden Analyte freies Prostata-spezifisches Antigen (fPSA) und mit $a_1$-Antichymotrypsin (ACT) komplexiertes Prostata-spezifisches Antigen (PSA-ACT) sind.

13. Verfahren nach Anspruch 12, wobei die aus der Probe zu bestimmenden Analyte weiterhin humanes Kallikrein 2 (hK2) als umfassen.

14. Verfahren nach einem Ansprüche 1 bis 11, wobei die aus der Probe zu bestimmenden Analyten Creatinkinease MB und Creaktinkinease BB sind.

15. Verfahren nach einem Ansprüche 1 bis 11, wobei die aus der Probe zu bestimmenden Analyten zumindest zwei Analyten umfassen, die aus Luteinisierendes Hormon (LH), Follikelstimulierendes Hormon (FSH), Thyreoidea-stimulierendes Hormon (TSH) und humanes Choriongonadotropin (hCG) ausgewählt sind.

16. Verfahren nach einem Ansprüche 1 bis 11, wobei die Analyten Protein-Isoformen sind.

17. Verfahren nach Anspruch 16, wobei die Analyten nicked hcG und non-nicked hCG sind.

18. Verfahren zum quantitativen Nachweis von fPSA, PSA-ACT und hK2, umfassend die Schritte

(a) Versetzen einer Probe, die fPSA, PSA-ACT und hK2 enthält, mit einem markierten Detektor-Bindepartner unter Bildung von Detektor-Analyt-Komplexen, die aus jeweils einem Analyt- und einem Detektormolekül bestehen;
(b) Anbinden der in Schritt (a) erhaltenen Detektor-Analyt-Komplexe an drei Fänger-Bindepartner unter Bildung von Detektor-Analyt-Fänger-Komplexen, wobei jeder Fänger-Bindepartner für einen Detektor-Analyten-Komplex spezifisch ist; und
(c) zeitabhängige Erfassung der Bildung der Detektor-Analyt-Fänger-Komplexe.

19. Verfahren zum quantitativen Nachweis von fPSA, PSA-ACT und hK2, umfassend die Schritte

(a) Versetzen einer Probe, die fPSA, PSA-ACT und hK2 enthält, mit einem markierten Detektor-Bindepartner unter Bildung von Detektor-Analyt-Komplexen, die aus jeweils einem Analyt- und einem Detektormolekül bestehen;
(b) Anbinden der in Schritt (a) erhaltenen Detektor-Analyt-Komplexe an drei Fänger-Bindepartner unter Bildung von Detektor-Analyt-Fänger-Komplexen, wobei einer der Fänger-Bindepartner für Detektor-fPSA-Komplexe spezifisch ist, ein weiterer der Fänger-Bindepartner für Detektor-hK2-Komplexe spezifisch ist und an einen dritten der Fänger-Bindepartner Detektor-fPSA-Komplexe und Detektor-PSA-ACT-Komplexe anbinden können; und
(c) zeitabhängige Erfassung der Bildung der Detektor-Analyt-Fänger-Komplexe.

**20.** Verfahren zum quantitativen Nachweis von fPSA und PSA-ACT, umfassend die Schritte

(a) Versetzen einer Probe, die fPSA und PSA-ACT enthält, mit einem markierten Detektor-Bindepartner unter Bildung von Detektor-Analyt-Komplexen, die aus jeweils einem Analyt- und einem Detektormolekül bestehen;
(b) Anbinden der in Schritt (a) erhaltenen Detektor-Analyt-Komplexe an zwei Fänger-Bindepartner unter Bildung von Detektor-Analyt-Fänger-Komplexen, wobei einer der Fänger-Bindepartner für Detektor-fPSA-Komplexe spezifisch ist und an einen der Fänger-Bindepartner Detektor-fPSA-Komplexe und Detektor-PSA-ACT-Komplexe anbinden können; und
(c) zeitabhängige Erfassung der Bildung der Detektor-Analyt-Fänger-Komplexe.

**Claims**

**1.** Method for the quantitative detection of a plurality of different analytes, the number n of analytes being at least 2, comprising the following steps:

(a) adding at least one labelled detector binding partner to a sample which contains the n analytes, in order to form detector-analyte complexes which respectively consist of an analyte molecule and at least one detector molecule, the number x of detector binding partners being less than or equal to n - 1, wherein each of the detector binding partners can bind to at least one analyte and at least one of the detector binding partners can bind to at least two analytes;
(b) binding the detector analyte complexes obtained in step (a) to capture binding partners in order to form detector-analyte-capture complexes, wherein the number y of capture binding partners corresponds to the number n of analytes and each capture binding partner is specific for at least one detector-analyte complex; and
(c) recording the formation of the detector-analyte-capture complexes as a function of time.

**2.** Method according to Claim 1, wherein at least one third binding partner which is specific for an analyte and can bind to a binding site of the analyte, which differs from the binding site to which the detector binding partners can bind, is added to the sample in step (a), wherein the detector-analyte complexes obtained in this way, which comprise the third binding partner, bind in step (b) via the third binding partner to the immobilized capture binding partners specific for the analyte.

**3.** Method according to one of the preceding claims, wherein at least one of the detector-analyte complexes can bind to two different capture binding partners.

**4.** Method according to one of the preceding claims, wherein the detector binding partner is labelled with a fluorescent or luminescent dye.

**5.** Method according to one of the preceding claims, wherein the detector binding partners, capture binding partners and third binding partners comprise ligands, polyclonal and monoclonal antibodies and their antigen-binding fragments; RNA; DNA; DNA/RNA derivatives and their analogues, for example aptamers; proteins, for example lectins; and allergens.

**6.** Method according to one of the preceding claims, wherein the capture binding partners are immobilized on different surface regions of a surface.

**7.** Method according to Claim 6, wherein the capture binding partners are bound to the surface directly via hydrophobic/ionic interactions or by chemical bonding.

**8.** Method according to Claim 6, wherein the capture binding partners are bound to the surface indirectly via immobilization mediators located on the surface, such as an anti-antibody, avidin or a carrier protein.

**9.** Method according to one of the preceding claims, wherein the recording of the formation of the detector-analyte-capture complexes as a function of time is carried out by measurements at least at two different times.

**10.** Method according to one of Claims 1 to 8, wherein the recording of the formation of the detector-analyte-capture complexes as a function of time is carried out at the end of the formation of the detector-analyte-capture complexes.

**11.** Method according to one of Claims 2 to 10, wherein the concentration of the detector-analyte-capture complexes is recorded by determining the increase in fluorescent light as a function of time.

**12.** Method according to one of the preceding claims, wherein the analytes to be determined from the sample are free prostate-specific antigen (fPSA) and prostate-specific antigen (PSA-ACT) complexed with $\square_1$-antichymotrypsin (ACT).

**13.** Method according to Claim 12, wherein the analytes to be determined from the sample furthermore comprise human kallikrein 2 (hK2).

**14.** Method according to one of Claims 1 to 11, wherein the analytes to be determined from the sample are creatine kinase MB and creatine kinase BB.

**15.** Method according to one of Claims 1 to 11, wherein the analytes to be determined from the sample comprise at least two analytes which are selected from luteinizing hormone (LH), follicle-stimulating hormone (FSH), thyroid-stimulating hormone (TSH) and human chorionic gonadotrophin (hCG).

**16.** Method according to one of Claims 1 to 11, wherein the analytes are protein isoforms.

**17.** Method according to Claim 16, wherein the analytes are nicked hCG and non-nicked hCG.

**18.** Method for the quantitative detection of fPSA, PSA-ACT and hK2, comprising the following steps:

(a) adding a labelled detector binding partner to a sample which contains fPSA, PSA-ACT and hK2, in order to form detector-analyte complexes which respectively consist of an analyte molecule and a detector molecule;
(b) binding the detector analyte complexes obtained in step (a) to capture binding partners in order to form detector-analyte-capture complexes, wherein each capture binding partner is specific for a detector-analyte complex; and
(c) recording the formation of the detector-analyte-capture complexes as a function of time.

**19.** Method for the quantitative detection of fPSA, PSA-ACT and hK2, comprising the following steps:

(a) adding a labelled detector binding partner to a sample which contains fPSA, PSA-ACT and hK2, in order to form detector-analyte complexes which respectively consist of an analyte molecule and a detector molecule;
(b) binding the detector analyte complexes obtained in step (a) to three capture binding partners in order to form detector-analyte-capture complexes, wherein one of the capture binding partners is specific for detector-fPSA complexes, another of the capture binding partners is specific for detector-hK2 complexes, and detector-fPSA complexes and detector-PSA-ACT complexes can bind to a third of the capture binding partners; and
(c) recording the formation of the detector-analyte-capture complexes as a function of time.

**20.** Method for the quantitative detection of fPSA and PSA-ACT, comprising the following steps:

(a) adding a labelled detector binding partner to a sample which contains fPSA and PSA-ACT, in order to form detector-analyte complexes which respectively consist of an analyte molecule and a detector molecule;
(b) binding the detector analyte complexes obtained in step (a) to two capture binding partners in order to form detector-analyte-capture complexes, wherein one of the capture binding partners is specific for detector-fPSA complexes, and detector-fPSA complexes and detector-PSA-ACT complexes can bind to one of the capture binding partners; and
(c) recording the formation of the detector-analyte-capture complexes as a function of time.

**Revendications**

**1.** Procédé pour la détermination quantitative de plusieurs analytes différents, le nombre n des analytes étant au moins égal à 2, comprenant les étapes suivantes

(a) addition à un échantillon, qui contient n analytes, d'au moins un partenaire de liaison-détecteur marqué, avec formation de complexes détecteur-analyte qui consistent chacun en une molécule d'analyte et au moins une molécule de détecteur, le nombre x des partenaires de liaison-détecteurs étant inférieur ou égal à n - 1, chacun des partenaires de liaison-détecteurs pouvant se lier à au moins un analyte et au moins un des partenaires de liaison-détecteurs pouvant se lier à au moins deux analytes ;

(b) liaison des complexes détecteur-analyte obtenus dans l'étape (a) à des partenaires de liaison-capteurs avec formation de complexes détecteur-analyte-capteur, le nombre y des partenaires de liaison-capteurs correspondant au nombre n des analytes et chaque partenaire de liaison-capteur étant spécifique d'au moins un complexe détecteur-analyte ; et

(c) détermination en fonction du temps de la formation des complexes détecteur-analyte-capteur.

2.  Procédé selon la revendication 1, dans lequel on ajoute à l'échantillon dans l'étape (a) au moins un troisième partenaire de liaison qui est spécifique d'un analyte et peut se fixer à un site de liaison de l'analyte qui diffère du site de liaison auquel peuvent se fixer les partenaires de liaison-détecteurs, les complexes détecteur-analyte ainsi obtenus, qui comprennent le troisième partenaire de liaison se liant dans l'étape (b), par l'intermédiaire du troisième partenaire de liaison, au partenaire de liaison-capteur spécifique de l'analyte, immobilisé.

3.  Procédé selon l'une quelconque des revendications précédentes, dans lequel au moins un des complexes détecteur-analyte peut se lier à deux partenaires de liaison-capteurs différents.

4.  Procédé selon l'une quelconque des revendications précédentes, dans lequel le partenaire de liaison-détecteur est marqué avec un colorant fluorescent ou luminescent.

5.  Procédé selon l'une quelconque des revendications précédentes, dans lequel les partenaires de liaison-détecteurs, partenaires de liaison-capteurs et troisièmes partenaires de liaison comprennent des ligands, des anticorps monoclonaux ou polyclonaux et leurs fragments se liant à l'antigène ; un ARN ; un ADN ; des dérivés ADN-ARN et leurs analogues, comme par exemple des aptamères, des protéines, comme par exemple des lectines ; et des allergènes.

6.  Procédé selon l'une quelconque des revendications précédentes, dans lequel les partenaires de liaison-capteurs sont immobilisés sur des zones superficielles distinctes d'une surface.

7.  Procédé selon la revendication 6, dans lequel les partenaires de liaison-capteurs sont fixés directement à la surface par des interactions hydrophobes/ioniques ou par liaison chimique.

8.  Procédé selon la revendication 6, dans lequel les partenaires de liaison-capteurs sont fixés indirectement à la surface par un agent provoquant l'immobilisation, se trouvant sur la surface, tel qu'un anticorps, l'avidine ou une protéine porteuse.

9.  Procédé selon l'une quelconque des revendications précédentes, dans lequel la détermination en fonction du temps de la formation des complexes détecteur-analyte-capteur s'effectue par des mesures à au moins deux instants différents.

10. Procédé selon l'une quelconque des revendications 1 à 8, dans lequel la détermination en fonction du temps de la formation des complexes détecteur-analyte-capteur s'effectue au point final de la formation des complexes détecteur-analyte-capteur.

11. Procédé selon l'une quelconque des revendications 2 à 10, dans lequel la concentration des complexes détecteur-analyte-capteur est déterminée par mesure de l'accroissement de lumière fluorescente en fonction du temps.

12. Procédé selon l'une quelconque des revendications précédentes, dans lequel les analytes à déterminer à partir de l'échantillon sont l'antigène spécifique de la prostate, libre (fPSA) et l'antigène spécifique de la prostate complexé avec l'antichymotrypsine (ACT) $a_1$.

13. Procédé selon la revendication 12, dans lequel les analytes à déterminer à partir de l'échantillon comprennent en outre la kallicréine 2 humaine (hK2) .

14. Procédé selon l'une quelconque des revendications 1 à 11, dans lequel les analytes à déterminer à partir de l'échantillon sont la créatine kinase MB et la créatine kinase BB.

15. Procédé selon l'une quelconque des revendications 1 à 11, dans lequel les analytes à déterminer à partir de l'échantillon comprennent au moins deux analytes qui sont choisis parmi l'hormone lutéinisante (LH), l'hormone folliculostimulante (FSH), la thyréostimuline (TSH) et la gonadotrophine chorionique humaine (hCG).

**16.** Procédé selon l'une quelconque des revendications 1 à 11, dans lequel les analytes sont des isoformes de protéines.

**17.** Procédé selon la revendication 16, dans lequel les analytes sont la hcG clivée ou la hCG non clivée.

**18.** Procédé pour la détermination quantitative de fPSA, PSA-ACT et hK2, comprenant les étapes suivantes

(a) addition à un échantillon, qui contient fPSA, PSA-ACT et hK2, d'au moins un partenaire de liaison-détecteur marqué, avec formation de complexes détecteur-analyte qui consistent chacun en une molécule d'analyte et une molécule de détecteur ;
(b) liaison des complexes détecteur-analyte obtenus dans l'étape (a) à trois partenaires de liaison-capteurs avec formation de complexes détecteur-analyte-capteur, chaque partenaire de liaison-capteur étant spécifique d'un complexe détecteur-analyte ; et
(c) détermination en fonction du temps de la formation des complexes détecteur-analyte-capteur.

**19.** Procédé pour la détermination quantitative de fPSA, PSA-ACT et hK2, comprenant les étapes suivantes

(a) addition à un échantillon, qui contient fPSA, PSA-ACT et hK2, d'un partenaire de liaison-détecteur marqué, avec formation de complexes détecteur-analyte qui consistent chacun en une molécule d'analyte et une molécule de détecteur ;
(b) liaison des complexes détecteur-analyte obtenus dans l'étape (a) à trois partenaires de liaison-capteurs avec formation de complexes détecteur-analyte-capteur, un des partenaires de liaison-capteurs étant spécifique des complexes détecteur-fPSA, un autre des partenaires de liaison-capteurs étant spécifique des complexes détecteur-hK2 et les complexes détecteur-fPSA et complexes détecteur-PSA-ACT pouvant se lier à un troisième des partenaires de liaison-capteurs ; et
(c) détermination en fonction du temps de la formation des complexes détecteur-analyte-capteur.

**20.** Procédé pour la détermination quantitative de fPSA et PSA-ACT, comprenant les étapes suivantes

(a) addition à un échantillon, qui contient fPSA et PSA-ACT, d'un partenaire de liaison-détecteur marqué, avec formation de complexes détecteur-analyte qui consistent chacun en une molécule d'analyte et une molécule de détecteur ;
(b) liaison des complexes détecteur-analyte obtenus dans l'étape (a) à deux partenaires de liaison-capteurs avec formation de complexes détecteur-analyte-capteur, un des partenaires de liaison-capteurs étant spécifique des complexes détecteur-fPSA et les complexes détecteur-fPSA et complexes détecteur-PSA-ACT pouvant se lier à l'un des partenaires de liaison-capteurs ; et
(c) détermination en fonction du temps de la formation des complexes détecteur-analyte-capteur.

Fig. 1

P = PSA
h = hK2
A = ACT
Ofb. = Oberflächenbereich

**Fig. 2a**

P = PSA
h = hK2
A = ACT
Ofb. = Oberflächenbereich

Fig. 2b

Fig. 3

17

7

8

Anti-⊗Ofb.

Anti-▷Ofb.

Anti-○Ofb.

9

12

11

10

P = PSA
h = hK2
A = ACT
Ofb. = Oberflächenbereich

18

14

15

Anti-⊗Ofb.

Anti-▷Ofb.

Anti-○Ofb.

9

12

11

10

**Fig. 4**

Zeitliche Zunahme des Fluoreszenzsignals durch Anbinden des Detektor-fPSA-Komplexes
beim Anbinden an zwei verschiedene Oberflächenbereiche

Fig. 5

EP 1 485 717 B1

Zeitliche Zunahme des Fluoreszenzsignals durch Anbinden des Detektor-PSA-ACT-Komplexes beim Anbinden an einen Oberflächenbereich

<u>Fig. 6</u>

EP 1 485 717 B1

**Kalibrationsfunktionen**

Fig. 7

EP 1 485 717 B1

Zeitliche Zunahme des Fluoreszenzsignals durch Anbinden von Detektor-fPSA- und Detektor-PSA-ACT-Komplexen beim Anbinden an zwei verschiedene Oberflächenbereiche

Fig. 8

PSA

freies PSA

hK2

humanes Kallikrein 2

ACT

PSA

PSA-ACT Komplex

**Fig. 9**